# EUROPEAN PATENT SPECIFICATION

(11) **EP 1 508 567 B1**
(45) Date of publication and mention of the grant of the patent: **15.11.2006**
(21) Application number: 04022598.9
(22) Date of filing: 23.09.1999
(51) Int. Cl.: C07C 253/30, C07C 255/57

(54) **Process for producing cyanobenzoic acid derivatives**
Verfahren zur Herstellung von Derivaten der Cyanobenzoesäure
Procédé de préparation de dérivés de l'acide cyano-benzoique

(30) Priority: 24.09.1998 JP 27021398; 09.10.1998 JP 28779698; 14.10.1998 JP 29196398; 23.10.1998 JP 30296098; 23.10.1998 JP 30296198; 17.11.1998 JP 32621198; 18.11.1998 JP 32817198; 15.04.1999 JP 10736599; 21.04.1999 JP 11362199
(43) Date of publication of application: 23.02.2005
(62) Divisional of application: 99118800.4
(73) Proprietor: SHOWA DENKO KABUSHIKI KAISHA, Minato-ku, Tokyo (JP)
(72) Inventor: Yasuda, Hiroshi Kawasaki Research Laboratory, Kawasaki-k Kawasaki-shi Kanagawa210-0858 (JP); Ito, Haruaki, Minato-ku Tokyo 105-8518 (JP); Tani, Takashi Kawasaki Works, Showa Denko K.K., Kawasaki-shi Kanagawa 210-0865 (JP); Ohshiro, Kimitaka Kawasaki Research Laboratory, Kawasaki-k Kawasaki-shi Kanagawa210-0858 (JP); Saito, Makoto Kawasaki Works, Showa Denko K.K., Kawasaki-shi Kanagawa 210-0865 (JP); Soya, Sumio, Minato-ku Tokyo 105-8518 (JP); Marumo, Kuniomi, Tokyo 157-0072 (JP)
(74) Representative: Strehl Schübel-Hopf & Partner

(56) References cited:
- WO-A-99/06360
- DE-A- 2 817 505
- US-A- 4 629 700
- DATABASE WPI Section Ch, Week 200006 Derwent Publications Ltd., London, GB; Class A41, AN 2000-072585 XP002158895 & WO 99/61411 A1 (SHOWA DENKO KK) 2 December 1999 (1999-12-02)
- DATABASE WPI, Section Ch, Week 200031, Derwent Publications Ltd., London, GB; Class A41, AN 2000-353368, XP002152733 & JP 2000 086609 A (SHOWA DENKO KK), 28 March 2000
- DATABASE WPI, Section Ch, Week 197719, Derwent Publications Ltd., London, GB; Class E14, AN 1977-33423Y, XP002158893 & JP 52039648 A (SANPO KAGAKU KENKUYUSHO KK), 28 March 1977 (1977-03-28) *abstract*
- DATABASE WPI, Section Ch, Week 200031, Derwent Publications Ltd., London, GB; Class B05, AN 2000-353369, XP002158894 & JP 2000 086610 A (SHOWA DENKO KK), 28 March 2000 (2000-03-28) *abstract*

## Description

### FIELD OF THE INVENTION

The present invention relates to a process for producing a cyanobenzoic acid compound.

The cyanobenzoic acid compound which is produced through the process according to the present invention is a useful intermediate for a variety of chemicals such as pharmaceuticals, agrochemicals, liquid crystals, and monomers for functional polymers.

### BACKGROUND OF THE INVENTION

Conventional processes for producing a cyanobenzoic acid compound will be described in the following.

JP-A-64-47, Dokl. Akad. Nauk. SSSR, 312, 5, 1129, (1990), and J. Organomet. Chem., 358, 1-3, 563, (1988) disclose processes involving reaction of a halobenzonitrile, such as chlorobenzonitrile, with carbon monoxide in the presence of a catalyst. SU 1467053 and Azerv. Khim. Zh., 1, 26, (1983) disclose processes involving oxidation of the methyl group of tolunitrile. U.S. Patent 4,213,917 and WO 9009975 disclose processes involving oxidation of the aldehyde group of cyanobenzaldehyde. JP-A-50-71643 and JP-A-50-83346 disclose processes involving disproportionation between a nitrile and a carboxylic acid. These processes also involve problems. For example, raw materials are difficult to obtain or expensive, and severe conditions are required. Thus, these methods are not industrially advantageous and are insufficient as low-cost methods.

Among cyanobenzoic acid compounds, p-cyanobenzoic acid is synthesized in a classic manner; i.e., Sandmeyer's reaction in which p-aminobenzoic acid is diazotized and the diazo species is reacted with copper cyanide (Lucas *et al*., J. Am. Chem. Soc., 51 (1929) 2718). Alternatively, processes for the synthesis involving oxidation of tolunitrile with a strong oxidizing agent such as chromic acid or permanganic acid (Levine et al., J. Org. Chem., 24 (1959) 115; and Kattwinkel *et al*., Chem. Ber., 37 (1904) 3226). These processes also involve problems. For example, Sandmeyer's reaction requires toxic copper cyanide; particularly, isolation and purification of p-cyanobenzoic acid under acidic conditions is difficult and dangerous due to generation of free hydrogen cyanide. Use of an oxidizing agent such as chromic acid or permanganic acid entails producing a toxic heavy metal waste as a by-product, which causes production of a large amount of toxic wastewater and thereby involves an environmental problem.

JP-A-61-85194 discloses that one nitrile group of terephthalonitrile is biologically hydrolyzed with an enzyme such as mononitrilase, to thereby synthesize p-cyanobenzoic acid. However, selectivity of hydrolysis of one nitrile group is poor, and hydrolysis must be carried out under low-concentration conditions, to thereby result in low productivity. In addition, by-products such as terephthalamic acid, terephthalamide, and terephthalic acid are inevitably produced along with the target p-cyanobenzoic acid.

Arkhipova *et al.*, J. Gen. Chem. USSR, 33 (1963) .631 disclose a process involving hydrolysis of one nitrile group of terephthalonitrile with pressurized aqueous ammonia. It is reported that one nitrile group of terephthalonitrile is hydrated to form p-cyanobenzamide, and the amido group thereof is further hydrolyzed to form p-cyanobenzoic acid. However, the above hydrolysis requires high temperature and pressure and is not preferred, in consideration of safety.

As described above, conventionally known techniques for producing p-cyanobenzoic acid disadvantageously involve problems such as poor operational safety, formation of by-products, and difficulty in attaining production of high-purity compounds.

### SUMMARY OF THE INVENTION

In view of the foregoing, the object of the present invention is to provide a process for producing a cyanobenzoic acid compound, which comprises the step of oxidizing a cyanobenzylamine compound.

Preferably, the cyanobenzylamine compound is oxidized in the presence of a ruthenium oxide compound or an iron oxide compound.

More preferably, the above-described oxidation step is carried out by use of an oxidizing agent other than a ruthenium oxide compound or an iron oxide compound, in the presence of the ruthenium oxide compound or the iron oxide compound.

Preferably, the oxidizing agent is a hypohalogeous acid compound or a persulfate salt compound.

Preferably, the above-described oxidation step is carried out in water or water containing an aprotic polar solvent.

Preferably, the above-described oxidation step is carried out at a pH of 7.5-12.

Preferably, the cyanobenzylamine compound is a compound represented by the following formula: 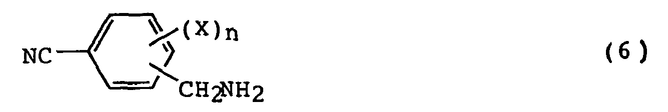 wherein each of -CH₂NH₂ and -X represents a substituent of the benzene ring; the -CH₂NH₂ group is bonded at the m- or the p-position with respect to the CN group; X represents a chlorine atom or a fluorine atom; n is an integer between 0 and 4 inclusive; and when n is 2 or more the plurality of X's may be identical to or different from one another,
and the cyanobenzoic acid compound is a compound represented by the following formula: 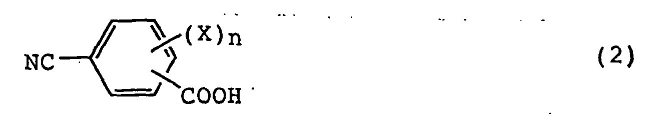 wherein -COOH, -X, and -CN have the same definitions as described above.

Preferably, the cyanobenzylamine compound represented by formula (6) is m- or p-cyanobenzylmaine, and the cyanobenzoic acid compound represented by formula (2) is m- or p-cyanobenzoic acid.

### DETAILED DESCRIPTION OF PREFERRED EMBODIMENTS

### Process for producing a cyanobenzoic acid compound from a cyanobenzylamine compound serving as a raw material

This process comprises reacting a cyanobenzylamine compound with an iron compound or a ruthenium compound, and with an oxidant (other than an iron compound or a ruthenium compound), and a base in accordance with need, in water or a mixture solvent of water and an aprotic polar solvent in a reaction vessel. The reaction proceeds with stirring at a predetermined temperature for a predetermined period of time.

The cyanobenzylamine compound used in the process is described below. Examples of unsubstituted cyanobenzylamine compounds include p-cyanobenzylamine and m-cyanobenzylamine, and these are synthesized by reduction of one nitrile group of terephthalonitrile and isophthalonitrile, respectively (JP-B-40-10133), and are easily obtained in large amounts.

Next, a description will be given of cyano-group-containing benzylamine compounds substituted by, for example, an alkyl group, an alkoxyl group, a halogen atom.

Cyanobenzylamine compounds substituted by halogen are described below. For example, chlorinated cyanobenzylamine compounds such as 4-cyano-2,3,5,6-tetrachlorobenzylamine and 3-cyano-2,4,5,6-tetrachlorobenzylamine are synthesized by reduction of one nitrile group of a chlorinated terephthalonitrile compound such as tetrachloroterephthalonitrile obtained by chlorination of terephthalonitrile, and one nitrile group of a chlorinated isophthalonitrile compound such as tetrachloroisophthalonitrile obtained by chlorination of isophthalonitrile. These chlorinated cyanobenzylamine compounds are easily obtained in large amounts. In addition, fluorinated cyanobenzylamine compounds such as 4-cyano-2,3,5,6-tetrafluorobenzylamine and 3-cyano-2,4,5,6-tetrafluorobenzylamine are synthesized by reduction of one nitrile group of a fluorinated terephthalonitrile compound such as tetrafluoroterephthalonitrile obtained by fluorination of a chlorinated terephthalonitrile compound such as tetrachloroterephthalonitrile, and one nitrile group of a fluorinated isophthalonitrile compound such as tetrafluoroisophthalonitrile obtained by fluorination of a chlorinated isophthalonitrile compound such as tetrachloroisophthalonitrile. These fluorinated cyanobenzylamine compounds are easily obtained in large amounts.

In the process, a ruthenium compound or an iron compound is used as an oxidation catalyst and an oxidant. A ruthenium compound such as ruthnium tetroxide (RuO₄) or an iron compound such as Na₂FeO₄ may be used alone for oxidation of a cyanobenzylamine compound. However, in consideration of cost and conservation of resources, an oxidant (hereinafter the term "an oxidant" refers to an oxidant other than an iron compound or a ruthenium compound) is preferably used for oxidation while an iron compound or a ruthenium compound is used as an oxidation catalyst. When an oxidant is used the process requires addition of a catalytic amount of a ruthenium compound or an iron compound.

When a ruthenium compound is used as an oxidation catalyst, an oxidant oxidizes a low-valence ruthenium compound such as ruthenium trichloride, to thereby form an active high-valence ruthenium compound. The high-valence ruthenium compound oxidizes a cyanobenzylamine compound to thereby produce a cyanobenzoic acid compound, and subsequently the high-valence ruthenium compound *per se* is reduced to the low-valence ruthenium compound. In the process, the oxidant repeatedly oxidizes the low-valence ruthenium compound into the active high-valence ruthenium compound, to thereby provide a catalyst cycle.

When an iron compound is used as an oxidation catalyst, an oxidant oxidizes a low-valence iron compound such as di- or tri-valent iron compound, to thereby form an active high-valence iron compound. The high-valence iron compound oxidizes a cyanobenzylamine compound to thereby produce a cyanobenzoic acid compound, and subsequently the high-valence iron compound *per se* is reduced to the low-valence iron compound. In the process, the oxidant repeatedly oxidizes the low-valence iron compound into the active high-valence iron compound, to thereby provide a catalyst cycle.

When an iron compound is used as an oxidation catalyst, an oxidant oxidizes a low-valence iron compound such as di- or tri-valent iron compound, to thereby form an active high-valence iron compound. The high-valence iron compound oxidizes a cyanobenzylamine compound to thereby produce a cyanobenzoic acid compound, and then the high-valence iron compound *per se* is reduced into the low-valence iron compound. In the process, the oxidant repeatedly oxidizes the low-valence iron compound into the active high-valence iron compound, to thereby provide a catalyst cycle.

Examples of ruthenium compounds which may be used as an oxidation catalyst in the process employing an oxidant include ruthenium inorganic salts such as ruthenium tetroxide, ruthenium trichloride, ruthenium tribromide, ruthenium triiodide, ruthenium hydroxide, ruthenium oxide, and ruthenium nitrosyl nitrate and ruthenium complexes such as ruthenium acetylacetonato and dodecacarbonyl triruthenium.

Examples of iron compounds which may be used as an oxidation catalyst in the process employing an oxidant include ferrous chloride, ferric chloride, ferrous bromide, ferric bromide, ferrous iodide, ferric iodide, ferrous oxide, ferric oxide, ferrosoferric oxide (Fe₃O₄), ferrous hydroxide, ferric hydroxide, iron (III) oxyhydroxide (FeO(OH)), ferrous sulfide, ferric sulfide, iron disulfide, ferrous sulfate, ferric sulfate, ferrous nitrate, ferric nitrate, iron carbonate, ferrous thiocyanate, ferric thiocyanate, ferrous acetate, ferrous oxalate, and ferric oxalate.

The mol ratio of ruthenium compound or iron compound, which may be used as an oxidation catalyst in the process employing an oxidant, to the cyanobenzylamine compound is preferably 0.001-0.05.

No particular limitation is imposed on the oxidant which may be used in the process, so long as the oxidant is capable of oxidizing a ruthenium compound or an iron compound. Examples of such oxidants include basic hypohalogenous acid compounds and persulfates.

Examples of hypohalogenous acid compounds which may be used in the process include hypohalogenous acids such as hypochlorous acid, hypobromous acid, and hypoiodous acid and hypohalogenous acid salts such as sodium hypochlorite, potassium hypochlorite, calcium hypochlorite, barium hypochlorite, sodium hypobromite, potassium hypobromite, sodium hypoiodite, and potassium hypoiodite. Examples of persulfates which may be used in the process include ammonium persulfate, sodium persulfate, and potassium persulfate.

The oxidant used in the process may be added in one portion at the beginning of the reaction, or may be gradually added so that the reaction does not proceed rapidly. The mol ratio of the oxidant to the cyanobenzylamine compound is preferably 3-6.

The process is performed in a basic atmosphere. In a basic atmosphere, reoxidation of a ruthenium compound or an iron compound may be performed at a sufficient rate. In the process, a cyanobenzoic acid compound is produced in accordance with oxidation of a cyanobenzylamine compound, and the reaction mixture has acidity, and therefore a base is added to the mixture in order to maintain the basicity. In the process, the required amount of the base may be added in one portion at the beginning of the reaction, or the base may be added successively during the reaction so as to maintain the basicity of the mixture.

The pH of the reaction mixture is described below. When the mixture is strongly basic, the cyano group of a cyanobenzylamine compound serving as a raw material and the cyano group of a cyanobenzoic acid compound serving as the target compound may decompose; whereas when the mixture is acidic, the reaction proceeds slowly and involves many side reactions. Therefore, the pH of the reaction is preferably 7.5-12.

Examples of bases which may be used in the process include alkali metal hydroxides and alkaline earth metal hydroxides such as lithium hydroxide, sodium hydroxide, potassium hydroxide, magnesium hydroxide, and calcium hydroxide; alkali metal bicarbonates such as sodium bicarbonate and potassium bicarbonate; alkali metal carbonates and alkaline earth metal carbonates such as lithium carbonate, sodium carbonate, potassium carbonate, magnesium carbonate, and calcium carbonate; and alkali and alkaline earth metal oxides such as magnesium oxide and calcium oxide. The amount of the base used for the above-described object depends on the type and the amount of an oxidant which coexists with the base, but the minimum amount is required in order to maintain the basicity of the reaction mixture throughout the entire process. When a hypochlorous acid compound is used as an oxidant, two types of bases are necessary, i.e., a base which is used for the formation of a basic hypochlorous acid compound from the hypochlorous acid compound, and a base which is used for the formation of a carboxylic acid salt from the produced cyanobenzoic acid compound. The mol ratio of monovalent base to cyanobenzylamine compound is preferably 3-6.

When a persulfate is used as an oxidant, two types of bases are required; i.e., a base which is used for formation of a sulfate from a hydrogensulfate produced by reduction of the persulfate, and a base which is used for formation of a carboxylic acid salt from the produced cyanobenzoic acid compound. The mol ratio of monovalent base to cyanobenzylamine compound is preferably 7-13.

The solvent used in the process is described below. The reaction may be performed in an aqueous solution. Moreover, a water-insoluble intermediate may be produced in accordance with oxidation of a cyanobenzylamine compound, and therefore an aprotic polar solvent is also used in the reaction in order to partially dissolve the intermediate contained therein. Consequently, the reaction may proceed effectively. Examples of aprotic polar solvents which may be used in the process include ethers such as dioxane and diglyme; amides such as dimethylformamide; sulfur-containing solvents such as dimethyl sulfoxide and sulfolane; and nitrile-solvents such as acetonitrile. In the present process, the aprotic polar solvent is used in an amount by weight of at least five times that of the cyanobenzylamine compound, and the solvent is used within a range where the solvent can be mixed with water. Preferably, the solvent is used in an amount of 5-20 times that of the cyanobenzylamine compound on a weight basis.

An excessively low reaction temperature causes the reaction to proceed slowly, whereas a high reaction temperature causes decomposition of the nitrile group of a cyanobenzylamine compound, and therefore the temperature is preferably 0-80°C, more preferably 10-50°C. The reaction time depends on the nature of the solvent employed, but preferably falls within a range of 10 minutes to 15 hours.

When a surplus of an oxidant such as a hypochlorous acid compound remains in the reaction mixtureafter completion of the reaction, in accordance with need, urea may be added into the mixture for decomposition of the surplus.

Isolation and purification of a cyanobenzoic acid compound are described below. After completion of the reaction, the cyanobenzoic acid compound exists in an aqueous solution in the form of a carboxylic acid salt. Depending on its type, the salt may dissolve or precipitate in the solution. The solubility of the cyanobenzoic acid compound in water is very low, and the compound precipitates in the solution by mere addition of an acid thereto. Therefore, the solution is simply filtered and washed with water and then dried, to thereby obtain the cyanobenzoic acid compound, and the purity of thus-produced compound reflects the purity of a cyanobenzylamine compound serving as a raw material.

### EXAMPLES

The present invention will next be described in more detail by way of examples, which should not be construed as limiting the invention thereto. Unless otherwise indicated, all parts, percents, ratios and the like are by weight.

In Examples 1 to 31 and 45 to 59, high performance liquid chromatographic analysis was carried out under the following conditions.

### HPLC Analysis Conditions

Column: Shodex (registered trademark: Showa Denko K.K.) DE-513L + precolumn
Column temperature: 40°C
Eluent: water/acetonitrile/acetic acid = 2250/750/15 (ml) sodium 1-octanesulfonate 6.45 g Flow rate 1 ml/minute
Detector: UV 254 nm

In Examples 32 to 44 and 60 to 65, gas chromatographic analysis was carried out under the following conditions.

### GC Analysis Conditions

Column: DB1 (J&W) (30m capillary column, inside diameter 0.32mm)
Carrier: helium 3 cc/minute
Split ratio: 40
Detector: FID
Analysis conditions: injection at 300°C 100°C (10 minutes) → 15°C/minute (elevation) → 280°C (8 minutes) detection at 300°C

### Example 1

A mixture containing p-cyanobenzylamine (13.2 g), water (50 g), and ferric chloride (0.2 g) was stirred, and a 14 wt.% aqueous solution (200 g) of sodium hypochlorite was added dropwise thereto at room temperature over two hours. The reaction mixture was further stirred for one hour. Subsequently, water (100 g) and urea (4 g) were added to the mixture, and the resultant mixture was further stirred for 20 minutes. The pH of the mixture was adjusted to four through addition of 98 wt.% sulfuric acid. The precipitated crystals were collected through filtration, washed with water, and dried, to thereby obtain 12.1 g of p-cyanobenzoic acid (yield 82%, based on p-cyanobenzylamine). High performance liquid chromatographic analysis revealed that the obtained p-cyanobenzoic acid had a purity of 95%.

### Example 2

A mixture containing p-cyanobenzylamine (13.2 g), water (50 g), and ruthenium trichloride (0.2 g) was stirred, and a 14 wt.% aqueous solution (200 g) of sodium hypochlorite was added dropwise thereto at room temperature over two hours. The reaction mixture was further stirred for one hour. Subsequently, water (100 g) and urea (4 g) were added to the mixture, and the resultant mixture was further stirred for 20 minutes. The pH of the mixture was adjusted to four through addition of 98 wt.% sulfuric acid. Precipitated crystals were collected through filtration, washed with water, and dried, to thereby obtain 10.4 g of p-cyanobenzoic acid (yield 76%, based on p-cyanobenzylamine). High performance liquid chromatographic analysis revealed that the p-cyanobenzoic acid obtained had a purity of 95%.

### Example 3

A mixture containing p-cyanobenzylamine (13.2 g), dioxane (70 g), sodium carbonate (5.3 g), water (50 g), and ferric nitrate (0.1 g) was stirred, and a 14 wt.% aqueous solution (200 g) of sodium hypochlorite was added dropwise thereto over three hours while inside temperature of a reactor was maintained at 50°C or lower. The reaction mixture was further stirred for two hours. Subsequently, water (100 g) and urea (4 g) were added to the mixture, and the resultant mixture was further stirred for 20 minutes. The pH of the mixture was adjusted to four through addition of 98 wt.% sulfuric acid. Precipitated crystals were collected through filtration, washed with water, and dried, to thereby obtain 11.9 g of p-cyanobenzoic acid (yield 90%, based on p-cyanobenzylamine). The p-cyanobenzoic-acid obtained had a purity of 96%.

### Example 4

A mixture containing p-cyanobenzylamine (13.2 g), dioxane (70 g), sodium carbonate (5.3 g), water (50 g), and ruthenium trichloride (0.1 g) was stirred, and a 14 wt.% aqueous solution (200 g) of sodium hypochlorite was added dropwise thereto over three hours while the inside temperature of a reactor was maintained at 50°C or lower. The reaction mixture was further stirred for two hours. Subsequently, water (100 g) and urea (4 g) were added to the mixture, and the resultant mixture was further stirred for 20 minutes. The pH of the mixture was adjusted to four through addition of 98 wt.% sulfuric acid. Precipitated crystals were collected through filtration, washed with water, and dried, to thereby obtain 13.5 g of p-cyanobenzoic acid (yield 92%, based on p-cyanobenzylamine). The p-cyanobenzoic acid obtained had a purity of 96%.

### Example 5

A mixture containing m-cyanobenzylamine (13.2 g), acetonitrile (80 g), sodium hydrogencarbonate (76 g), water (500 g), sodium persulfate (95 g), and iron oxide (0.25 g) was stirred for reaction at 80°C over ten hours. After the precipitated solid was removed through filtration, the pH of the filtrate was adjusted to 4 through addition of sulfuric acid. The precipitated crystals were collected through filtration, washed with water, dried, to thereby obtain 7.1 g of m-cyanobenzoic acid (yield 48%, based on m-cyanobenzylamine). The m-cyanobenzoic acid obtained had a purity of 94%.

### Example 6

A mixture containing m-cyanobenzylamine (13.2 g), acetonitrile (80 g), sodium hydrogencarbonate (76 g), water (500 g), sodium persulfate (95 g), and ruthenium oxide (0.25 g) was stirred for reaction at 80°C over ten hours. After the precipitated solid was removed through filtration, the pH of the filtrate was adjusted to four through addition of sulfuric acid. The precipitated crystals were collected through filtration, washed with water, and dried, to thereby obtain 8.2 g of m-cyanobenzoic acid (yield 56%, based on m-cyanobenzylamine). The m-cyanobenzoic acid obtained had a purity of 94%.

## Claims

1. A process for producing a cyanobenzoic acid compound, which comprises oxidizing a cyanobenzylamine compound.

2. A process for producing a cyanobenzoic acid compound according to Claim 1, wherein the oxidizing is carried out by the use of a oxidizing agent.

3. A process for producing a cyanobenzoic acid compound according to Claim 2, wherein the mole ratio of the oxidizing agent to the cyanobenzylamine compound is 3 - 6.

4. A process for producing a cyanobenzoic acid compound according to any one of Claims 1 to 3, wherein the oxidizing is carried out in the presence of a ruthenium oxide compound or an iron oxide compound.

5. A process for producing a cyanobenzoic acid compound according to any one of Claims 1 to 4, wherein the oxidizing is carried out by the use of an oxidizing agent other than a ruthenium oxide compound or an iron oxide compound, in the presence of the ruthenium oxide compound or the iron oxide compound.

6. A process for producing a cyanobenzoic acid compound according to Claim 5, wherein the mole ratio of the ruthenium oxide compound or iron oxide compound to the cyanobenzylamine compound is 0.001 - 0.05.

7. A process for producing a cyanobenzoic acid compound according to Claim 5 or 6, wherein the oxidizing agent is a hypohalogeous acid compound or a persulfate salt compound.

8. A process for producing a cyanobenzoic acid compound according to any one of Claims 1 to 7, wherein the oxidizing is carried out in water or water containing an aprotic polar solvent.

9. A process for producing a cyanobenzoic acid compound according to any one of Claims 1 to 8, wherein the oxidizing is carried out at a pH of 7.5-12.

10. A process for producing a cyanobenzoic acid compound according to any of Claims 1 to 9, wherein the oxidizing is carried out at a temperature of 0 - 80°C.

11. A process for producing a cyanobenzoic acid compound according to any one of Claims 1 to 10, wherein the cyanobenzylamine compound is a compound represented by the following formula 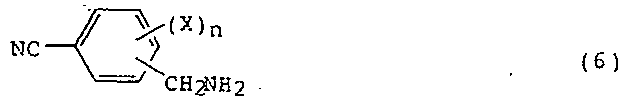 wherein each of -CH₂NH₂ and -X represents a substituent of the benzene ring; the -CH₂NH₂ group is bonded at the m- or the p-position with respect to the CN group; X represents a chlorine atom or a fluorine atom; n is an integer between 0 and 4 inclusive; and when n is 2 or more the plurality of X's may be indentical to or different from one another; and the cyanobenzoic acid compound is a compound represented by the following formula: 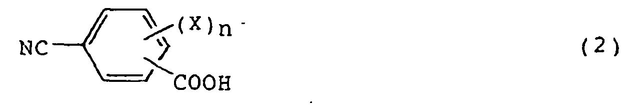 wherein -COOH, -X, and -CN have the same definitions as described above.

12. A process for producing a cyanobenzoic acid compound according to Claim 11, wherein the cyanobenzylamine compound represented by formula (6) is m- or p-cyanobenzylamine, and the cyanobenzoic acid compound represented by formula (2) is m- or p-cyanobenzoic acid.

## Patentansprüche

1. Verfahren zum Herstellen einer Cyanobenzoesäureverbindung, welches die Oxidation einer Cyanobenzylaminverbindung umfasst.

2. Verfahren zum Herstellen einer Cyanobenzoesäureverbindung nach Anspruch 1, wobei die Oxidation unter Einsatz eines Oxidationsmittels durchgeführt wird.

3. Verfahren zum Herstellen einer Cyanobenzoesäureverbindung nach Anspruch 2, wobei das Molverhältnis des Oxidationsmittels zu der Cyanobenzylaminverbindung 3 bis 6 ist.

4. Verfahren zum Herstellen einer Cyanobenzoesäureverbindung nach einem der Ansprüche 1 bis 3, wobei die Oxidation in Anwesenheit einer Rutheniumoxidverbindung oder einer Eisenoxidverbindung durchgeführt wird.

5. Verfahren zum Herstellen einer Cyanobenzoesäureverbindung nach einem der Ansprüche 1 bis 4 , wobei die Oxidation unter Einsatz eines anderen Oxidationsmittels als einer Rutheniumoxidverbindung oder einer Eisenoxidverbindung in Anwesenheit einer Rutheniumoxidverbindung oder Eisenoxidverbindung durchgeführt wird.

6. Verfahren zum Herstellen einer Cyanobenzoesäureverbindung nach Anspruch 5, wobei das Molverhältnis der Rutheniumoxidverbindung oder Eisenoxidverbindung zu der Cyanobenzylaminverbindung 0,001 bis 0,05 ist.

7. Verfahren zum Herstellen einer Cyanobenzoesäureverbindung nach Anspruch 5 oder 6, wobei das Oxidationsmittel eine Verbindung einer hypohalogenigen Säure oder eine Persulfatsalzverbindung ist.

8. Verfahren zum Herstellen einer Cyanobenzoesäureverbindung nach einem der Ansprüche 1 bis 7, wobei die Oxidation in Wasser oder in ein aprotisches polares Lösungsmittel enthaltendem Wasser durchgeführt wird.

9. Verfahren zum Herstellen einer Cyanobenzoesäureverbindung nach einem der Ansprüche 1 bis 8, wobei die Oxidation bei einem pH-Wert von 7,5 bis 12 durchgeführt wird.

10. Verfahren zum Herstellen einer Cyanobenzoesäureverbindung nach einem der Ansprüche 1 bis 9, wobei die Oxidation bei einer Temperatur von 0 bis 80°C durchgeführt wird.

11. Verfahren zum Herstellen einer Cyanobenzoesäureverbindung nach einem der Ansprüche 1 bis 10, wobei die Cyanobenzylaminverbindung eine Verbindung der folgenden Formel 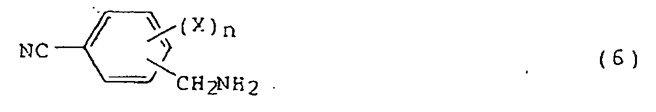 ist, worin -CH₂NH₂ und -X jeweils einen Substituenten des Benzolringes darstellen; die -CH₂NH₂-Gruppe bezüglich der CN-Gruppe an der m- oder der p-Position gebunden ist; X ein Chloratom oder ein Fluoratom darstellt; n eine ganze Zahl zwischen 0 und einschließlich 4 ist; und wenn n 2 oder mehr ist, können die Substituenten X jeweils gleich oder unterschiedlich sein; und die Cyanobenzoesäureverbindung ist eine Verbindung der folgenden Formel: 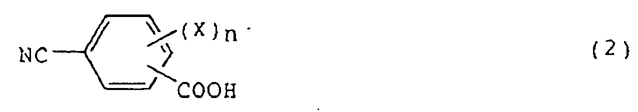 worin -COOH, -X und -CN dieselben Bedeutungen wie vorstehend beschrieben haben.

12. Verfahren zum Herstellen einer Cyanobenzoesäureverbindung nach Anspruch 11, wobei die Cyanobenzylaminverbindung der Formel (6) m- oder p-Cyanobenzylamin ist und die Cyanobenzoesäureverbindung der Formel (2) m- oder p-Cyanobenzoesäure ist.

## Revendications

1. Procédé de production d'un composé d'acide cyanobenzoïque, qui comprend l'oxydation d'un composé de cyanobenzylamine.

2. Procédé de production d'un composé d'acide cyanobenzoïque selon la revendication 1, dans lequel l'oxydation est effectuée grâce à l'utilisation d'un agent oxydant.

3. Procédé de production d'un composé d'acide cyanobenzoïque selon la revendication 2, dans lequel le rapport molaire de l'agent oxydant sur le composé de cyanobenzylamine est égal à 3 - 6.

4. Procédé de production d'un composé d'acide cyanobenzoïque selon l'une quelconque des revendications 1 à 3, dans lequel l'oxydation est effectuée en présence d'un composé d'oxyde de ruthénium ou d'un composé d'oxyde de fer.

5. Procédé de production d'un composé d'acide cyanobenzoïque selon l'une quelconque des revendications 1 à 4, dans lequel l'oxydation est effectuée grâce à l'utilisation d'un agent oxydant autre qu'un composé d'oxyde de ruthénium ou d'un composé d'oxyde de fer, en présence du composé de l'oxyde de ruthénium ou du composé d'oxyde de fer.

6. Procédé de production d'un composé d'acide cyanobenzoïque selon la revendication 5, dans lequel le rapport molaire du composé d'oxyde de ruthénium ou du composé d'oxyde de fer sur le composé de cyanobenzylamine est égal à 0,001 - 0,05.

7. Procédé de production d'un composé d'acide cyanobenzoïque selon la revendication 5 ou 6, dans lequel l'agent oxydant est un composé d'acide hypohalogéneux ou un composé de sel de persulfate.

8. Procédé de production d'un composé d'acide cyanobenzoïque selon l'une quelconque des revendications 1 à 7, dans lequel l'oxydation est effectuée dans de l'eau ou dans de l'eau contenant un solvant polaire aprotique.

9. Procédé de production d'un composé d'acide cyanobenzoïque selon l'une quelconque des revendications 1 à 8, dans lequel l'oxydation est effectuée à un pH de 7,5 - 12.

10. Procédé de production d'un composé d'acide cyanobenzoïque selon l'une quelconque des revendications 1 à 9, dans lequel l'oxydation est effectuée à une température de 0 - 80 °C.

11. Procédé de production d'un composé d'acide cyanobenzoïque selon l'une quelconque des revendications 1 à 10, dans lequel le composé de cyanobenzylamine est un composé représenté par la formule suivante : 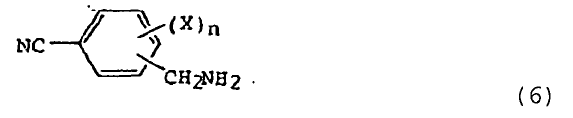 où chacun de -CH₂NH₂ et -X représente un substituant de l'anneau benzénique ; le groupe -CH₂NH₂ est lié en position m- ou p- par rapport au groupe CN ; X représente un atome de chlore ou un atome de fluor ; n est un nombre entier entre 0 et 4, inclus ; et lorsque n est 2, ou un chiffre supérieur, la pluralité des X peuvent être identiques entre eux ou différents les uns des autres ;
et le composé d'acide cyanobenzoïque est un composé représenté par la formule suivante : 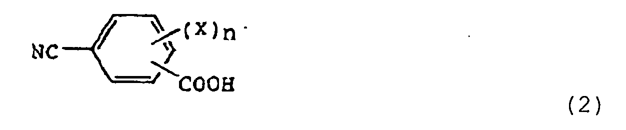 où -COOH, -X et -CN ont les mêmes définitions que celles décrites ci-dessus.

12. Procédé de production d'un composé d'acide cyanobenzoïque selon la revendication 11, dans lequel le composé de cyanobenzylamine représenté par la formule (6) est du m- ou p-cyanobenzylamine, et le composé d'acide cyanobenzoïque représenté par la formule (2) est l'acide m- ou p-cyanobenzoïque.
